# EUROPEAN PATENT SPECIFICATION

(11) **EP 2 303 361 B1**
(45) Date of publication and mention of the grant of the patent: **25.02.2015**
(21) Application number: 09766276.1
(22) Date of filing: 17.06.2009
(51) Int. Cl.: A61M 5/142, A61M 31/00

(54) **CAPSULE FOR DELIVERY OF POWDER LIKE MEDICATION IN THE GASTRO-INTESTINAL TRACT**
KAPSEL ZUR ABGABE VON PULVERFÖRMIGEM MEDIKAMENT IN DEN VERDAUUNGSTRAKT
CAPSULE POUR L'ADMINISTRATION DE MÉDICAMENT SOUS FORME DE POUDRE DANS L'APPAREIL DIGESTIF

(30) Priority: 19.06.2008 US 73778 P
(43) Date of publication of application: 06.04.2011
(73) Proprietor: Medimetrics Personalized Drug Delivery B.V., 5656 AE, Eindhoven (NL)
(72) Inventor: ZOU, Hans, NL-5656 AE Eindhoven (NL); SHIMIZU, Jeff, NL-5656 AE Eindhoven (NL); ALBU, Lucian, R., NL-5656 AE Eindhoven (NL); DIJKSMAN, Johan, F., NL-5656 AE Eindhoven (NL)
(74) Representative: Flügel Preissner Kastel Schober
(86) International application number: PCT/IB2009/052575
(87) International publication number: WO 2009/153739

(56) References cited:
- WO-A-01/52923
- DE-A1- 3 317 536
- US-A1- 2005 234 431
- US-A1- 2007 250 045

## Description

### FIELD OF THE INVENTION

The invention relates to a device for placement in an environment for delivery of medication to said environment.

### BACKGROUND OF THE INVENTION

In WO 94/07562 delivery capsules are disclosed which include a beneficial agent and an activating mechanism, which delivery capsules are designed to deliver the beneficial agent in a pulsatile manner through the orifice. The pulsatile delivery is achieved by a pair of guide members inside the capsule, one secured to the capsule itself and the other to a movable partition.

Techniques disclosed in WO 94/07562 have limited ability to deliver a powder like medication in a humid environment to said environment since the techniques have no facility to annul a solidification of the powder like medication induced by humidification.

A device according to the preamble of claim 1 is known from DE 3317536 A1.

### OBJECT OF THE INVENTION

It is an object of the invention defined in claim 1 to provide a device for a more reliable delivery of a medication in a humid environment.

### SUMMARY OF THE INVENTION

The object of the invention is achieved by the device according to the invention which comprises a reservoir having an orifice, a conveying unit for conveying a reservoir's content through the orifice and an actuator arrangement for driving the conveying unit, wherein the conveying unit comprises an auger extending in the reservoir, characterized in that the conveying unit comprises a collapsible barrier for pressurizing the reservoir's content and a grid, installed around the auger, to which the collapsible barrier is conformable.

By extending the auger in the reservoir, mechanical interaction with the reservoir's content is arrived at. As a result of the presence of at least a portion of the auger in the reservoir, in addition to conveying the powder like medication to the orifice of the reservoir, a solidification of a powder like medication contained in the reservoir through humidification in a moist environment or by way of a humidity already contained in the powder, is annulled through pulverization. Here, solidification of the powder like medication ranges from a wet paste to a solid. It is noted that the actuator arrangement can include one or more actuators known per se, such as a spring actuator, an electromagnetic actuator, a hydraulic actuator or a piezoelectric actuator. The auger can be made from a biocompatible plastic or a stainless steel.

Not according to the invention, the conveying unit may comprise a piston for pressurizing the reservoir's content. With that, under the pressure provided by the piston, the reservoir's content is continuously fed into an auger's helical flighting. As a result, a revolution of the auger eventuates at a well defined amount of powder like medication conveyed through the orifice.

Further, the conveying unit may be provided with a facility for pretensioning the piston to the reservoir's content. As a result, a continuous energizing of the actuator arrangement for pressurizing the reservoir's content, is prevented from.

Moreover, the piston may be driveable by the auger. As a result, the prerequisite for a further actuator to drive the piston is circumvented.

A surface profile of the piston may matche a profile of a reservoir's surface adjacent to or surrounding the orifice. As a result the residual reservoir's content is minimized. With that the yield of a capsule provided with this embodiment according to the invention is maximized.

According to the invention, however, the conveying unit comprises a collapsible barrier for pressurizing the reservoir's content. The collapsible barrier is conformable to a curved surface of revolution enveloping the auger. By selecting the dimensions of the curved surface of revolution enveloping the auger to be sufficiently small, a residual reservoir's content is minimized. With that a yield of a capsule provided with this embodiment according to the invention is maximized.

In a further embodiment according to the invention, the auger has an axially varying helical flighting height. Herein the helical flighting height is defined as a radius measured from an auger's axis of revolution to a curved surface of revolution enveloping the auger.

In an embodiment according to the invention, the axially varying helical flighting height increases with an axial distance measured from the orifice. As a result, an axially oriented pressure gradient can be exerted by the auger on the reservoir's content contained within a volume established by a revolution of the auger. The helical flighting height preferably monotonically increases with the distance from the orifice. Proximal to the orifice, the helical flighting height matches a size of the orifice with the purpose of continuation of the auger up to and including the reservoir's orifice.

In an embodiment according to the invention the auger has an axially varying helical flighting pitch. In this text, the helical flighting pitch is defined as an axial distance covered by a complete turn of the helical flighting

In a further embodiment according to the invention the helical flighting pitch increases with the axial distance measured from the orifice. As a result, an axially oriented pressure gradient can be exerted by the auger on the reservoir's content contained within the volume established by a revolution of the auger. Preferably, the helical flighting pitch monotonically increases with the distance from the orifice.

In a further embodiment according to the invention the device comprises a measuring apparatus for measuring a quantity of the reservoir's content conveyed through the orifice. Based on a measurement acquired by way of the measuring apparatus, the actuator arrangement can be controlled. As a result, a more accurate dosing of powder like medication to an environment is obtained.

In an embodiment according to the invention the measuring apparatus comprises a revolution counter for counting a number of revolutions made by the auger. Assuming that the helical flighting height and the helical flighting pitch are both known, the number of revolutions made by the auger provides an accurate measurement for the amount of the reservoir's content conveyed through the orifice.

A capsule according to the invention is defined in claim 10. The capsule can be made of any suitable material, such as a biocompatible plastic. Apart from being nonreactive to a medication or an acid, a biocompatible plastic material has a density which is larger than water which will ensure the capsule neither to float above water nor to be detained inside a gastrointestinal tract.

The invention is highly suitable for application in the field of targeted and precisely controlled delivery of a medication, especially a powder like medication, to an environment, particularly a humid environment, which environment may be inside a human or an animal body. A likely environment is a gastrointestinal tract.

### BRIEF DESCRIPTION OF THE DRAWINGS

The invention and its advantages are further elucidated by way of example with reference to the drawings in which:
Figure 1 schematically displays an embodiment of the device according to the invention, wherein a piston is driveable by an actuator arrangement.
Figure 2 schematically shows an embodiment of the device according to the invention, wherein a collapsible barrier is arranged for pressurizing a reservoir's content.
Figure 3 schematically depicts an embodiment of the device according to the invention, in which a piston is arranged for pressurizing a reservoir's content, wherein a spring facility serves for pretensioning the piston.
Figure 4 schematically shows an embodiment of the device according to the invention, wherein a piston is driveable by an auger.
Figure 5 schematically displays an embodiment of the device according to the invention, wherein a piston's surface profile matches a reservoir's surface profile surrounding an orifice.

### DETAILED DESCRIPTION OF THE EMBODIMENTS

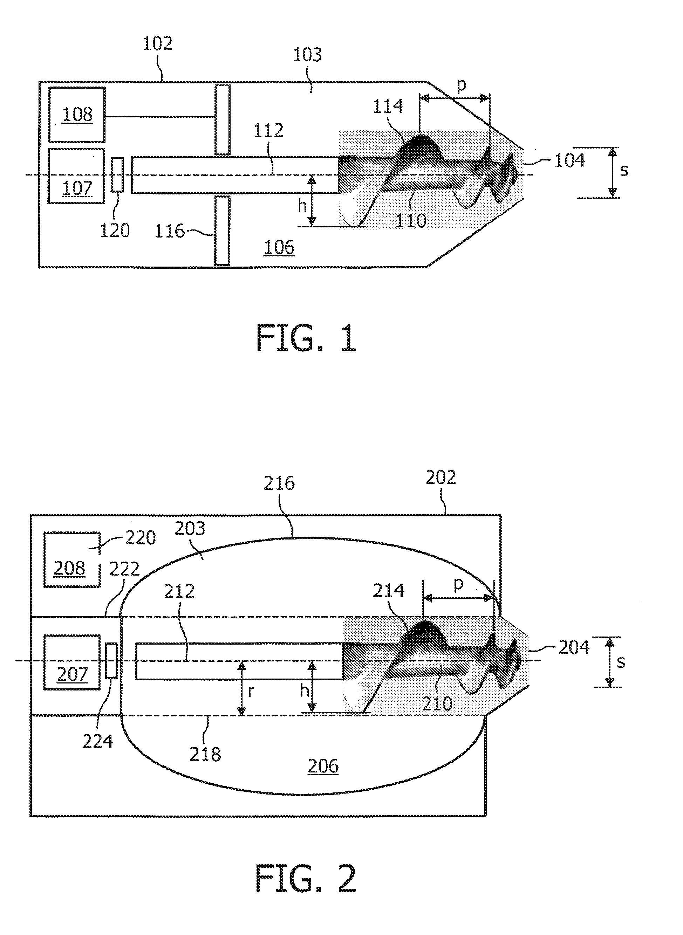

Figure 1 displays an examplary device in the form of a capsule 102 comprising a reservoir 103. The capsule 102 is at made of a biocompatible plastic, such as a medical grade polyethylene. The reservoir 103 is provided with an orifice 104. The orifice 104 can be provided with a hydrophobic coating as to minimize a diffusion into the reservoir 103 of a humidity present in a moist environment surrounding the capsule 102. The reservoir 103 contains a reservoir's content 106 which is a powder like medication. An actuator arrangement comprises an actuator 107 and a further actuator 108. The actuator 107, which is a spring actuator, an electromagnetic actuator, a hydraulic actuator or a piezoelectric actuator, causes an auger 110 to rotate around an axis of revolution 112 during use. The auger 110 is made of a biocompatible plastic or a stainless steel and extends into the reservoir 103 in order to provide a mechanical interaction with the reservoir's content 106 with the purpose of both conveying the reservoir's content 106 to the orifice 104 and annulling a solidification of the reservoir's content 106. The solidification of the reservoir's content 106 is caused by way of a humidification in the moist environment surrounding the capsule 102 or by way of a humidity already contained in the reservoir's content 106. The solidification of the reservoir's content 106 is annulled by way of pulverization through a revolution of the auger 110 during use. The auger 110 is provided with a helical flighting 114. The helical flighting has a helical flighting height h which monotonically increases with an axial distance from the orifice 104. Proximal to the orifice 104, the helical flighting height h matches a size s of the orifice with the purpose of continuation of the auger 110 up to and including the orifice 104. In addition to that, the helical flighting 114 has a helical flighting pitch p which monotonically increases with an axial distance measured from the orifice 104. As a result an axially oriented pressure gradient can be exerted on the reservoir's content 106 contained within a volume, which volume is established by a revolution of the auger 110. A piston 116 serves for pressurizing the reservoir's content 106. With that, under a pressure provided by the piston 116 during use, the reservoir's content 106 is continuously fed into the helical flighting 114 of the auger 110. The piston 116, which is made of a biocompatible plastic or a stainless steel and is optionally provided with a non-sticky coating, is driveable by the further actuator 108. The further actuator 108 is an electromagnetic actuator or a hydraulic actuator. A measurement apparatus 120, comprising a revolution counter, serves for measuring a number of revolutions made by the auger 110. A measurement signal generated by the measurement apparatus 120 serves for controlling the actuator 107 and the further actuator 108. Since the helical flighting height h and the helical flighting pitch p are both known, the number of revolutions made by the auger 110 provides a measurement for the amount of the reservoir's content 106 conveyed through the orifice 104. For the purpose of a high auger transportation efficiency, an inside surface of the reservoir 103 can be provided with a non-sticky coating whereas the helical flighting 114 of the auger 110 can be provided with certain roughness features.

Figure 2 displays an embodiment according to the invention in the form of a capsule 202 comprising a reservoir 203. The capsule 202 is made of a biocompatible plastic. The reservoir 203 is provided with an orifice 204. The reservoir 203 contains a reservoir's content 206 which is a powder like medication. An actuator arrangement 207 comprises an actuator 207 and a further actuator 208. The actuator 207, which is a spring actuator, an electromagnetic actuator, a hydraulic actuator or a piezoelectric actuator, causes an auger 210 to revolute during use around an axis of revolution 212. The auger 210 is provided in the reservoir 203 in order to provide mechanical interaction with the reservoir's content 206 with the purpose of both conveying the reservoir's content 206 to the orifice 204 and annulling a solidification of the reservoir's content 206. The auger 210 is provided with a helical flighting 214. The helical flighting has a helical flighting height h which monotonically increases with an axial distance from the orifice 204. Proximal to the orifice 204, the helical flighting height h matches a size s of the orifice with the purpose of continuation of the auger 210 up to and including the orifice 204. In addition to that, the helical flighting 214 has a helical flighting pitch p which monotonically increases with an axial distance from the orifice 204. A collapsible barrier 216 for pressurizing the reservoir's content 206 is contained in the reservoir 203. With that, under the pressure provided by the collapsible barrier 216, the reservoir's content 206 is continuously fed into the helical flighting 214 of the auger 210. The collapsible barrier 216 is conformable to a tube like grid 218 installed around the auger 210 which tube like grid 218 is to prevent the collapsible barrier 216 from contacting the auger 210. By choosing a radius r of the grid 218 minimally, i.e. by choosing it only slightly larger than the maximum level of the helical flighting height h, a residue of the reservoir's content 206 is minimized. The collapsible barrier 216 is compressible by the further actuator 208. The further actuator 208 comprises a cartridge 220 that releases an inert gas upon actuation in the volume established by the reservoir 203, the collapsible barrier 216 and a seal 222 as to provide a pressure to the collapsible barrier 216. A measurement apparatus 224 comprising a revolution counter, serves for measuring a number of revolutions made by the auger 210. A measurement signal generated by the measurement apparatus 224 serves for controlling the actuator 207 and the further actuator 208.

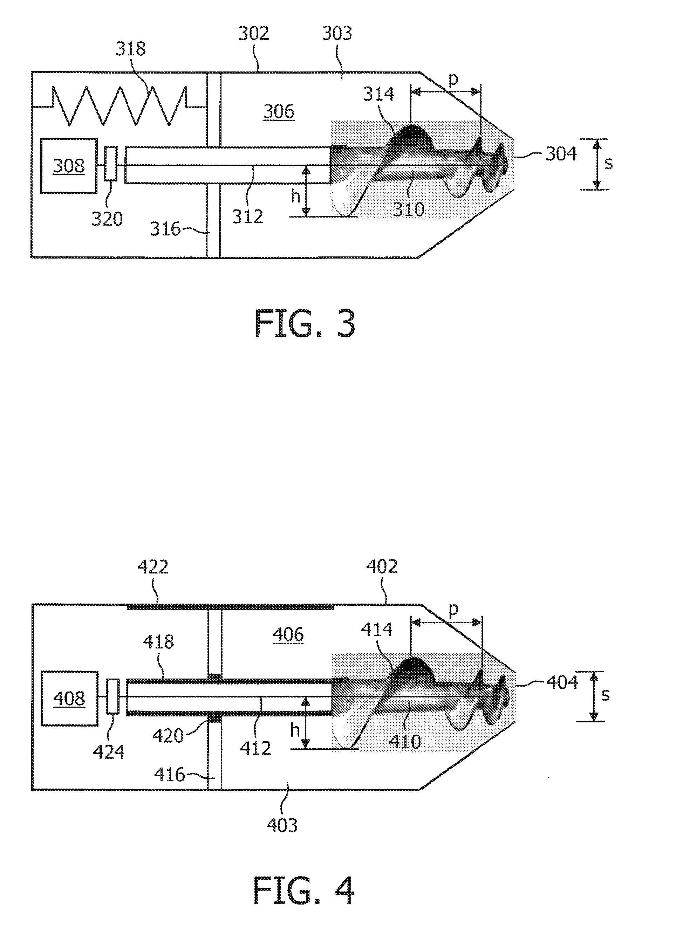

Figure 3 displays another exemplary device in the form of a capsule 302 comprising a reservoir 303. The reservoir is provided with an orifice 304. The reservoir 303 contains a reservoir's content 306 which is a powder like medication. An actuator arrangement comprises an actuator 308. The actuator 308 causes an auger 310 to revolute around an axis of revolution 312 during use. The auger 310 is present in the reservoir 303 in order to provide a mechanical interaction with the reservoir's content 306 with the purpose of both conveying the reservoir's content 306 to the orifice 304 and annulling a solidification of the reservoir's content 306. The auger 310 is provided with a helical flighting 314. The helical flighting has a helical flighting height h which increases with an axial distance from the orifice 304. Proximal to the orifice 304, the helical flighting height h matches a size s of the orifice with the purpose of continuation of the auger 310 up to and including the orifice 304. In addition to that, the helical flighting 314 has a helical flighting pitch p which increases with an axial distance from the orifice 304. As a result an axially oriented pressure gradient can be exerted on the reservoir's content 306 contained within a volume which volume is established by a revolution of the auger 310. A piston 316 serves for pressurizing the reservoir's content 306. With that, under a pressure provided by the piston 316 during use, the reservoir's content 306 is continuously fed into the helical flighting 314 of the auger 310. The piston 316 is pretensioned to the reservoir's content 306 by way a facility for pretensioning. The facility for pretensioning comprises an elastic mechanical spring 318. The facility for pretensioning the piston 316 to the reservoir's content 306 may alternatively comprise an air spring, a repulsive magnetic element or a combination thereof. Trough this, a continuous energizing of an actuator to provide a pressure to the reservoir's content 304 is prevented from. A measurement apparatus 320 comprising a revolution counter, serves for measuring a number of revolutions made by the auger 310. A measurement signal generated by the measurement apparatus 320 serves for controlling the actuator 308.

Figure 4 displays a further exemplary device in the form of a capsule 402 comprising a reservoir 403. The reservoir is provided with an orifice 404. The orifice 404 can be provided with a hydrophobic coating as to minimize a diffusion into the reservoir 403 of a humidity present in a moist environment surrounding the capsule 402. The reservoir 403 contains a reservoir's content 406 which is a powder like medication. An actuator arrangement comprises an actuator 408. The actuator 408, which is a spring actuator, an electromagnetic actuator, a hydraulic actuator or a piezoelectric actuator, causes an auger 410 to rotate around an axis of revolution 412 during use. The auger 410 extends into the reservoir 403 with the purpose of providing a mechanical interaction with the reservoir's content 406. The auger 410 is provided with a helical flighting 414. The helical flighting has a helical flighting height h which increases with an axial distance from the orifice 404. Proximal to the orifice 404, the helical flighting height h matches a size s of the orifice with the purpose of continuation of the auger 110 up to and including the orifice 404. In addition to that, the helical flighting 414 has a helical flighting pitch p which increases with an axial distance from the orifice 404. A piston 416 serves for pressurizing the reservoir's content 406. The piston 416 for pressurizing the reservoir's content 406 is driveable by the auger 410. For driving the piston 416, the auger 410 is supplied with an auger screw thread 418 for cooperation with a piston screw thread 420. A rail 422 is supplied to the reservoir 402 to accurately guide a displacement of the piston 416 and to prevent the piston 416 from rotating with the auger 410. A measurement apparatus 424 comprising a revolution counter is arranged for measuring a number of revolutions made by the auger 410. A measurement signal generated by the measurement apparatus 424 serves for controlling the actuator 408.

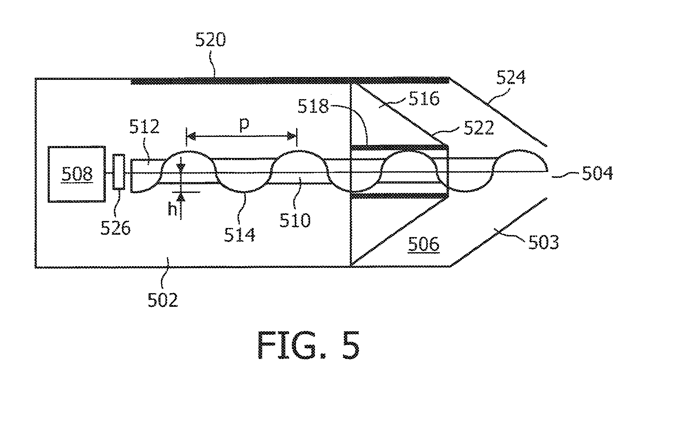

Figure 5 displays an example in the form of a capsule 502 comprising a reservoir 503. The reservoir 503 is provided with an orifice 504. The reservoir 503 contains a reservoir's content 506 which is a powder like medication. An actuator arrangement comprises an actuator 508. The actuator 508 causes an auger 510 to revolute around an axis of revolution 512 during use. The auger 510 extends in the reservoir 503 in order to provide a mechanical interaction with the reservoir's content 506 with the purpose of both conveying the reservoir's content 506 to the orifice 504 and annulling a solidification of the reservoir's content 506. The auger 510 is provided with a helical flighting 514. A piston 516 for pressurizing the reservoir's content 506 is driveable by the auger 510. With that, under a pressure provided by the piston 516 during use, the reservoir's content 506 are continuously fed into the helical flighting 514 of the auger 510. For driving the piston 516, the auger 510 is supplied with a constant helical flighting height h and a constant helical flighting pitch p. The helical flighting 514 is in cooperation with a piston screw thread 518. A rail 520 is supplied to the reservoir 502 to accurately guide a displacement of the piston 516 and to prevent the piston 516 from rotating with the auger 510. By driving the piston 516 through the auger 510, the prerequisite for a further actuator to drive the piston 516 is circumvented. The piston 516 has a surface profile 522 that corresponds to a reservoir's surface profile 524 adjacent to the orifice 504. Consequently, the piston 516 is transportable as far as the reservoir's orifice 504. As a result, a residue of the reservoir's content 506 is minimized. A measurement apparatus 526 comprising a revolution counter is arranged counting a number of revolutions made by the auger 510. A measurement signal generated by the measurement apparatus 526 is serving for controlling the actuator 508.

While the invention has been illustrated and described in detail in the foregoing description with respect to figure 2, the illustrations and the description are to be considered illustrative or exemplary. The invention is defined by the set of claims.

## Claims

1. A medical device for placement in an environment for delivery of medication to said environment, comprising a reservoir (103) having an orifice (104), a conveying unit for conveying a reservoir's content (106, 206, 306, 406, 506) through the orifice and an actuator arrangement for driving the conveying unit, wherein the conveying unit comprises an auger (110, 210, 310, 410, 510) extending in the reservoir, **characterized in that** the conveying unit comprises a collapsible barrier (216) for pressurizing the reservoir's content and a tube-like grid (218), installed around the auger, to which the collapsible barrier is conformable.

2. The device according to claim 1 comprising a cartridge (220) for pressurizing the collapsible barrier (216) upon release of an inert gas.

3. The device according to claim 1, wherein the auger has an axially varying helical flighting (114, 214, 314, 414, 514) height.

4. The device according to claim 3, wherein the axially varying helical flighting height increases with an axial distance from the orifice.

5. The device according to claim 4, wherein a radius of the grid (218) is only slightly larger than a maximum level of the axially varying helical flighting height.

6. The device according to claim 1, wherein the auger has an axially varying helical flighting pitch.

7. The device according to claim 6, wherein the axially varying helical flighting pitch monotonically increases with the axial distance from the orifice.

8. The device according to claim 1, comprising a measuring apparatus (120, 224, 320, 424, 526) for measuring a quantity of the reservoir's content conveyed through the orifice.

9. The device according to claim 8, wherein the measuring apparatus comprises a revolution counter for counting a number of revolutions made by the auger.

10. A capsule (102, 202, 302, 402, 502) provided with the device according to claim 1.

## Patentansprüche

1. Medizinische Vorrichtung zum Platzieren in einer Umgebung für die Abgabe von Medikamenten in die Umgebung, umfassend einen Behälter (103), der eine Öffnung (104) hat, eine Transporteinheit zum Transportieren des Inhalt des Behälters (106, 206, 306, 406, 506) durch die Öffnung und eine Aktoranordnung zum Antreiben der Transporteinheit, wobei die Transporteinheit eine Förderschnecke (110, 210, 310, 410, 510) umfasst, die sich in dem Behälter erstreckt, **dadurch gekennzeichnet, dass** die Transporteinheit eine faltbare Grenzschicht (216) zum Unterdrucksetzen des Inhalts des Behälters, und ein rohrförmiges Gitter (218) umfasst, das um die Förderschnecke verbaut ist, zu der die faltbare Grenzschicht formanpassfähig ist.

2. Vorrichtung nach Anspruch 1, umfassend eine Kartusche (220) zum Unterdrucksetzen der faltbaren Grenzschicht (216) nach Abgabe eines inerten Gases.

3. Vorrichtung nach Anspruch 1, wobei die Förderschnecke eine sich axial verändernde schneckenförmige Gewinde- (114, 214, 314, 414, 514) Höhe hat.

4. Vorrichtung nach Anspruch 3, wobei sich die axial verändernde schneckenförmige Gewindehöhe mit einer axialen Entfernung zur Öffnung erhöht.

5. Vorrichtung nach Anspruch 4, wobei ein Radius des Gitters (218) nur geringfügig größer ist als ein maximales Niveau der sich axial verändernden schneckenförmigen Gewindehöhe.

6. Vorrichtung nach Anspruch 1, wobei die Öffnung eine sich axial verändernde schneckenförmige Gewindeganghöhe hat.

7. Vorrichtung nach Anspruch 6, wobei sich die axial verändernde schneckenförmige Gewindeganghöhe gleichmäßig mit der axialen Entfernung zur Öffnung erhöht.

8. Vorrichtung nach Anspruch 1, umfassend eine Messeinrichtung (120, 224, 320, 424, 526) zum Messen einer Menge des Inhalts des Behälters, die durch die Öffnung transportiert wurde.

9. Vorrichtung nach Anspruch 8, wobei die Messeinrichtung einen Umdrehungszähler zum Zählen einer Anzahl von Umdrehungen umfasst, die von der Förderschnecke gemacht wurden.

10. Kapsel (102, 202, 302, 402, 502), die mit der Vorrichtung nach Anspruch 1 ausgestattet ist.

## Revendications

1. Dispositif médical à mettre en place dans un environnement pour la distribution d'un médicament audit environnement, comprenant un réservoir (103) ayant un orifice (104), une unité de convoyage pour convoyer un contenu (106, 206, 306, 406, 506) du réservoir à travers l'orifice et un agencement actionneur pour entraîner l'unité de convoyage, dans lequel l'unité de convoyage comprend une vis d'Archimède (110, 210, 310, 410, 510) s'étendant dans le réservoir, **caractérisé en ce que** l'unité de convoyage comprend une barrière capable de s'effondrer (216) pour mettre sous pression le contenu du réservoir, et une grille semblable à un tube (218) installée autour de la vis d'Archimède, sur laquelle la barrière capable de s'effondrer est susceptible de se conformer.

2. Dispositif selon la revendication 1, comprenant une cartouche (220) pour mettre sous pression la barrière capable de s'effondrer (216) lors de la libération d'un gaz inerte.

3. Dispositif selon la revendication 1, dans lequel la vis d'Archimède a une hauteur de spire hélicoïdale (114, 214, 314, 414, 514) qui varie axialement.

4. Dispositif selon la revendication 3, dans lequel la hauteur de spire hélicoïdale qui varie axialement augmente avec la distance axiale depuis l'orifice.

5. Dispositif selon la revendication 4, dans lequel un rayon de la grille (218) est uniquement légèrement plus grand qu'un niveau maximum de la hauteur de spire hélicoïdale qui varie axialement.

6. Dispositif selon la revendication 1, dans lequel la vis d'Archimède possède un pas de spire hélicoïdale qui varie axialement.

7. Dispositif selon la revendication 6, dans lequel le pas de spire hélicoïdale qui varie axialement augmente de manière monotone avec la distance axiale depuis l'orifice.

8. Dispositif selon la revendication 1, comprenant un appareil de mesure (120, 224, 320, 424, 526) pour mesurer une quantité du contenu du réservoir convoyé à travers l'orifice.

9. Dispositif selon la revendication 8, dans lequel l'appareil de mesure comprend un compteur de rotations pour compter un nombre de rotations effectuées par la vis d'Archimède.

10. Capsule (102, 202, 302, 402, 502) dotée du dispositif selon la revendication 1.
